# DEMANDE DE BREVET EUROPEEN

(11) **EP 4 763 184 A1**
(43) Date de publication de la demande: **24.06.2026**
(21) Numéro de dépôt: 25217231.7
(22) Date de dépôt: 20.11.2025
(51) Int. Cl.: A61K 8/04, A61K 8/34, A61K 8/60, A61K 8/81, A61Q 19/00

(54) **GEL AQUEUX, SON UTILISATION EN COSMÉTIQUE ET PROCÉDÉ POUR DIMINUER L'EFFET COLLANT RESSENTI LORS DE SON APPLICATION**

(30) Priorité: 25.11.2024 FR 2412950
(71) Demandeur: Société d'Exploitation de Produits pour les Industries Chimiques SEPPIC, 75321 Paris cedex 07 (FR)
(72) Inventeur: CAMBOS, Sophie, 81100 Castres (FR); ROSO, Alicia, 81100 Castres (FR); LAUBE, Florian, 81100 Castres (FR)
(74) Mandataire: Air Liquide

(57) **Abrégé**

Gel aqueux comprenant de 0,5% massique à 5,0% massique d'un polyélectrolyte anionique réticulé (PA) comportant de 5,0% molaire à 95,0% molaire, d'unités monomériques issues de d'acide 2-méthyl-2-((1-oxo-2-propènyl) amino)-1-propanesulfonique partiellement ou totalement salifié sous forme de sel alcalin ou de sel d'ammonium, de 4,9% molaire à 90,0% molaire d'unités monomériques de N,N-dialkylacrylamide et de 0,1% molaire à 5,0% molaire d'unités monomériques issues d'un monomère de formule CH₂=C(CH₃)-C(=O)-O-(CH₂-CH₂-O-)ₙ-R₅ ; de 0,5% massique à 9,5% massique, soit d'un alcanol de formule R₁-OH, ou d'un mélange d'alcanols de formules R₁-OH; soit d'une composition (C) d'alkyl polyglycosides représentés par les formules R₁-O-(G)ₓ-H, dans lesquelles G représente ou bien le reste du glucose ou bien le reste du xylose et x représente un nombre décimal compris entre 1,05 et 2,05 ; soit d'un mélange (M) dudit alcanol de formule R₁-OH ou dudit mélange d'alcanols de formules R₁-OH et de ladite composition (C) ; de 0,0% massique à 3,0% massique un ou plusieurs actifs cosmétiques et la quantité nécessaire en eau pour compléter à 100% massique dans lequel le rapport massique (R_{M}) ayant comme numérateur la masse du ou des alcanols de formule R₁-OH, ou la masse de ladite composition (C) ou la masse dudit mélange (M) et comme dénominateur, la masse dudit polyélectrolyte anionique réticulé (PA), est supérieur ou égal à 1 et inférieur ou égal à 10 ; procédé de soin cosmétique de la peau mettant en oeuvre ledit gel aqueux ; utilisation, soit dudit alcanol de formule R₁-OH, ou d'un mélange d'alcanols de formules R₁-OH, soit de ladite composition (C) soit dudit mélange (M), en vue de réduire ou d'éliminer l'effet collant ressenti lors de l'application sur la peau d'un gel aqueux comprenant ledit un polyélectrolyte anionique (PA) ; procédé pour réduire ou éliminer l'effet collant ressenti lors de l'application sur la peau d'un gel aqueux comprenant ledit polyélectrolyte anionique (PA) en modifiant sa composition avant son application sur la peau, par ajout soit du ou des alcanols de formule R₁-OH, soit de ladite composition (C), soit dudit mélange (M).

## Description

L'invention concerne, de façon générale, le domaine de la cosmétique et plus particulièrement le domaine des formulations cosmétiques topiques.

L'invention se rapporte plus spécifiquement à l'amélioration de propriétés sensorielles de gels aqueux. Elle a ainsi pour objet un nouveau gel aqueux, optionnellement riche en sels, son utilisation en tant que composition cosmétique à usage topique, ainsi qu'un nouveau procédé pour diminuer l'effet collant d'un gel aqueux épaissi, optionnellement riche en sels.

### Etat de la technique antérieure

Les sérums sont largement utilisés aujourd'hui pour le traitement cosmétique de la peau. Par sérum, on désigne des solutions aqueuses, qui ont pour objectif d'être absorbées rapidement par les couches superficielles de l'épiderme, et qui souvent contiennent une concentration élevée en ingrédients actifs. Ils sont généralement utilisés en complément aux différentes formulations de soin de la peau pour en augmenter l'effet.

Un tel traitement comprend une première étape de nettoyage de la peau, puis une deuxième étape d'application du sérum sur la peau nettoyée et enfin une dernière étape d'application de la formulation de soin cosmétique souhaitée. Ce procédé est particulièrement pertinent lorsque l'utilisateur constate que l'effet de la formulation de soin est diminué, en raison de facteurs externes comme les conditions climatiques ou environnementales, ou en cas de changement brutal de l'état de la peau comme par exemple son assèchement.

Les sérums comprennent des substances présentant des propriétés diverses, comme des propriétés hydratantes, éclaircissantes, purifiantes, anti-âge, antirides, énergisantes, anticernes ou anti-sébum.

Les sérums cosmétiques sont généralement des gels aqueux dans lesquels les substances actives sont maintenues en suspension ou solubilisées de façon à ce qu'elles soient appliquées directement sur la peau. De la sorte, les possibilités d'interférence avec d'autres ingrédients, comme les huiles, sur la peau sont surmontées.

Pour que les sérums puissent être rapidement absorbés par le *stratum corneum*, ils doivent présenter une texture non grasse, et un équilibre entre des caractéristiques d'écoulement suffisamment fluides pour être rapidement étalés sur la surface de la peau à traiter ; ils doivent aussi être suffisamment gélifiés pour éviter les coulures au moment de l'application.

Les polymères synthétiques et les polymères d'origine naturelle sont des agents épaississants de phases aqueuses cosmétiques bien connus de l'homme du métier et qui permettent de préparer un gel aqueux pour des utilisations topiques dans le domaine de la cosmétique et de la pharmacie.

Les polymères épaississants synthétiques actuellement utilisés dans ce domaine, se présentent sous forme de poudre ou sous forme liquide en tant qu'une émulsion eau-dans-huile du polymère préparé par polymérisation radicalaire en émulsion inverse à l'aide d'agents tensioactifs, couramment dénommée latex inverse. Parmi les polymères épaississants synthétiques en poudre les plus connus, on peut citer les polymères à base d'acide acrylique ou de ses esters comme ceux commercialisés sous les noms, Carbopol^{™} ou Pemulen^{™}. Ils sont décrits notamment dans les brevets américains US5373044, US2798053 ainsi que dans la demande de brevet européen EP0301532A2. Il y a aussi les polymères à base d'acide 2-acrylamido-2-méthyl propanesulfonique et de ses sels comme ceux commercialisés sous le nom Aristoflex^{™}. Ils sont décrits notamment dans les brevets européens EP0816403, EP1116733 et EP1069142. Ces poudres sont généralement obtenues par polymérisation précipitante du (ou des) monomère(s) en solution dans un solvant organique type benzène, acétate d'éthyle, cyclohexane ou tertio-butanol ; ce procédé nécessite donc de nombreuses étapes successives de purification du produit final, pour éliminer toute trace de solvant résiduel.

Parmi les latex inverses les plus connus on peut citer ceux commercialisés sous les noms Sepigel^{™}, Simulgel^{™}, Sepiplus^{™} et Sepilife^{™}. Ces épaississants sont obtenus par polymérisation radicalaire en émulsion inverse. Ils sont plus aisément manipulables, notamment à température ambiante, et se dispersent très rapidement dans l'eau. De plus, ils développent des performances épaississantes remarquablement élevées. Cependant, comme ils contiennent au moins une huile, cela les rend potentiellement moins pertinents pour préparer des gels aqueux sans possibilité d'interférence entre les actifs et la peau.

Il existe aussi des épaississants synthétiques ayant les performances épaississantes équivalentes ou supérieures aux latex inverses, mais présentant des possibilités d'usage plus polyvalentes, en particulier du fait de l'absence de toute phase huile pouvant conduire à des gels aqueux. Ils se présentent sous la forme de poudres mais possèdent des temps de dissolution, et donc une facilité de mise en oeuvre, comparable à ceux des produits se présentant sous la forme de liquides. Ils sont décrits dans la demande de brevet européen publiée sous le numéro EP1496081A2. Ils sont obtenus par les techniques classiques de polymérisation, telles que la polymérisation radicalaire en phase dispersée, la polymérisation radicalaire en suspension inverse, la polymérisation radicalaire en émulsion inverse ou en microémulsion inverse combinés suivies d'une séparation par différentes techniques telles que la précipitation dans un tiers solvant, la précipitation dans un tiers solvant puis éventuellement d'un lavage, d'un séchage par atomisation ou par déshydratation azéotropique, suivie éventuellement d'un lavage par un solvant judicieusement choisi. Ces épaississants synthétiques combinent donc une partie des avantages des épaississants synthétiques se présentant sous la forme de poudres classiques (absence d'huile, obtention de gels aqueux plus clairs) et les avantages des épaississants synthétiques se présentant sous la forme de latex inverse (pré-neutralisation, haute vitesse de dissolution, pouvoir épaississant et propriétés stabilisantes remarquables).

Cependant, les gels obtenus avec ces épaississants synthétiques n'ont pas une stabilité satisfaisante lorsque la composition est riche en électrolytes comme c'est souvent le cas des sérums comprenant par exemple des extraits de végétaux riches en électrolytes, ou bien des sels minéraux.

Des terpolymères linéaires, branchés ou réticulés d'au moins un monomère possédant une fonction acide fort, libre, partiellement salifiée ou totalement salifiée, et au moins un monomère de formule CH₂=C(CH₃)-C(=O)-O-(CH₂-CH₂-O-)ₙ-R₂, dans laquelle R₂ représente un radical alkyle linéaire ou ramifié comportant de huit à vingt atomes de carbone et n représente un nombre supérieur ou égal à 1 et inférieur ou égal à 30, sont décrits dans la demande internationale publiée sous le numéro WO2011030044.

Cependant, lorsque des gels aqueux sont préparés par la mise en oeuvre de tels terpolymères épaississants synthétiques en présence d'électrolytes, les consommateurs ressentent une sensation collante après application sur la peau. Cette sensation est désagréable et nécessite un temps d'attente avant l'application d'un autre produit de soin qui finalise la routine. En effet, de façon générale, le sérum est appliqué avant la crème de soin et par conséquent, son application sur la peau ne doit pas conférer à celle-ci un caractère collant.

La demande internationale de brevet publiée sous le numéro WO2018024962A1, décrit la préparation de gels aqueux comprenant des polyélectrolytes anioniques réticulés de formule (A), des sels, des espèces électrolytiques, de l'eau, et des esters de sorbitan, tels que les oléates de sorbitan éthoxylés qui présentent notamment une faible turbidité et une grande stabilité au stockage à température ambiante et à 45°C. Cependant, de tels gels aqueux présentent des caractéristiques collantes sur la peau qui ne sont pas encore satisfaisantes et demandent à être améliorées.

Une autre solution peut consister en l'ajout d'huiles de silicone dans le gel aqueux mais les huiles de silicone sont abandonnées pour la préparation de formulations cosmétiques car elles ne sont pas suffisamment biodégradables.

Une autre solution consiste à associer des alcanes biodégradables au gel aqueux en s'inspirant de l'enseignement technique contenu dans la demande internationale de brevet numéro WO2018109353. Cependant, si leur apport est pertinent dans le cadre des émulsions huile-dans-eau, de tels alcanes confère au gel aqueux un état sensoriel gras et huileux non désiré.

### Exposé de l'invention

Il est donc apparu nécessaire de développer un gel aqueux qui montre un aspect homogène après trois mois de stockage à une température de 20°C ainsi qu'à 45°C, qui ne procure pas une sensation collante après son application sur la peau et qui ne comprend pas d'huile pour éviter toute interférence avec les couches supérieures de l'épiderme.

C'est pourquoi, selon un premier aspect, l'invention a pour objet un gel aqueux caractérisé en ce qu'il comprend, pour 100% de sa masse :
- de 0,5% massique à 5,0% massique d'un polyélectrolyte anionique réticulé (PA) comportant pour 100% molaire :
   - de 5,0% molaire à 95,0% molaire, d'unités monomériques issues de l'acide 2-méthyl-2-((1-oxo-2-propènyl) amino)-1-propanesulfonique de formule (I) (CH₂=CH-C(=O)-NH)-C(CH₃)₂-S(=O)₂-OH, partiellement ou totalement salifié sous forme de sel alcalin ou de sel d'ammonium,
   - de 4,9% molaire à 90,0% molaire d'unités monomériques issues d'un monomère de formule (II) CH₂=CH-C(=O)-N(R₄)₂, dans laquelle R₄ représente un radical alkyl linéaire ou ramifié comportant de un à quatre atomes de carbone, et
   - de 0,1% molaire à 5,0% molaire d'unités monomériques issues d'un monomère de formule (III) CH₂=C(CH₃)-C(=O)-O-(CH₂-CH₂-O-)ₙ-R₅, dans laquelle R₅ représente un radical alkyle linéaire ou ramifié comportant de huit à vingt atomes de carbone et n représente un nombre supérieur ou égal à 1 et inférieur ou égal à 20,
- de 0,5% massique à 9,5% massique :
   - soit d'un alcanol de formule (IV) R₁-OH, dans laquelle R₁ représente un radical alkyle linéaire ou ramifié comportant de sept à vingt-deux atomes de carbone, ou un mélange d'alcanols de formules (IV) ;
   - soit d'une composition (C) d'alkyl polyglycosides représentés par les formules (V) R₁-O-(G)ₓ-H, dans lesquelles G représente ou bien un reste du glucose ou bien un reste du xylose et x représente un nombre décimal compris entre 1,05 et 2,05, ladite composition (C) consistant, pour 100% molaire, en un mélange de a₁% molaire du composé de formule R₁-O-(G)₁-H, a₂% molaire du composé de formule
      R₁-O-(G)₂-H, a₃% molaire du composé de formule R₁-O-(G)₃-H, a₄% molaire du composé de formule R₁-O-(G)₄-H, et a₅% molaire du composé de formule R₁-O-(G)₅-H, tel que la somme a₁% + a₂% + a₃% +a₄% + a₅% est égale à 100% molaire et que la somme a₁% + 2a₂% + 3a₃% + 4a₄% + 5a₅% est égale à 100x ;
   - soit d'un mélange (M) dudit alcanol de formule (IV) ou dudit mélange d'alcanols de formules (IV) et de ladite composition (C),
- de 0,0% massique à 3,0% massique un ou plusieurs actifs cosmétiques choisis dans le groupe constitué par le rétinol, la vitamine C, le niacinamide (vitamine B3), l'undécylénoyl phénylalanine, l'acide glycolique, l'acide azélaïque, l'acide hyaluronique, le mélange de xylitol, de polyglucosides et de 1,4-anhydro xylitol et l'urée, et
- la quantité nécessaire en eau pour compléter à 100% massique, et en ce qu'au sein dudit gel aqueux, le rapport massique (R_{M}) ayant comme numérateur ou bien la masse dudit alcanol de formule (IV) ou dudit mélange d'alcanols de formules (IV), ou bien la masse de ladite composition (C) ou bien la masse dudit mélange (M) et comme dénominateur, la masse dudit polyélectrolyte anionique réticulé (PA), est supérieur ou égal à 1,0 et inférieur ou égal à 10,0.

Par polyélectrolyte anionique réticulé (PA), on désigne au sens de la présente invention, un polyélectrolyte anionique réticulé non linéaire, se présentant à l'état de réseau tridimensionnel insoluble dans l'eau, mais gonflable à l'eau et conduisant à l'obtention d'un gel chimique. Dans ce même cadre, l'agent de réticulation mis en œuvre dans la réaction polymérisation de laquelle est issu ledit polyélectrolyte anionique réticulé (PA), est un composé diéthylénique ou polyéthylènique plus particulièrement choisi parmi le diméthacrylate d'éthylèneglycol, le tétraallyloxyéthane, le diacrylate d'éthylèneglycol, le diallyl urée, le triallyl amine, le propanetriacrylate de triméthylole, le méthylène-bis(acrylamide) ou un mélange de ces composés. L'agent de réticulation est utilisé dans cette réaction en une proportion molaire exprimée par rapport aux monomères mis en œuvre, supérieure ou égale à 0,005% et inférieure ou égale à 1,000% molaire, plus particulièrement supérieure ou égale à 0,010% et inférieure ou égale à 0,500% molaire et tout particulièrement supérieure ou égale à 0,010% et inférieure ou égale à 0,250% molaire. Selon la concentration en sel du milieu aqueux constitutif dudit sérum cosmétique topique, ledit sérum cosmétique topique comprend généralement pour 100% de sa masse de 0,5% massique à 5,0% massique dudit polyélectrolyte anionique (PA).

Selon un premier mode particulier, le gel aqueux tel que défini ci-dessus est caractérisé en ce que ledit rapport massique (R_{M}) est supérieur ou égal à 1,5 et inférieur ou égal à 5,0

Selon un autre mode particulier le gel aqueux tel que défini ci-dessus est caractérisé en ce que ledit polyélectrolyte anionique réticulé (PA) comporte pour 100% molaire, de 60,0% molaire à 80,0% molaire d'unités monomérique issues de l'acide de formule (III), partiellement salifié sous forme de sel d'ammonium, de 15,0% molaire à 39,5% molaire d'unités monomériques issues du N,N-diméthyl acrylamide, et de 0,5% molaire à 5,0% molaire d'unités monomériques issues du méthacrylate de lauryle tétra-éthoxylé. Comme exemple d'un tel polyélectrolyte réticulé anionique (PA), il y a le SEPIMAX ZEN^{™} commercialisé par la société SEPPIC, qui un terpolymère réticulé au propanetriacrylate de triméthylol, comprenant pour 100% molaire, 77,4% molaire d'unités monomériques issues du 2-méthyl-2-((1-oxo-2-propényl) amino) 1-propanesulfonate d'ammonium, 19,2% molaire d'unités monomériques issues du N,N-diméthyl acrylamide et 3,4% molaire d'unités monomériques issues du méthacrylate de lauryle tétra-éthoxylé.

Selon un autre mode particulier le gel aqueux tel que défini ci-dessus est caractérisé en ce que dans l'eau constitutive dudit gel aqueux, est dissout au moins un sel hydrosoluble choisi dans le groupe constitué par les sels de métaux alcalins, les sels de métaux alcalino-terreux et les sels de métaux de transition, de sorte que la concentration totale au sein de l'eau en ledit au moins un sel hydrosoluble, est supérieure à 0,00 mole par litre et inférieure ou égale à 1,00 mole par litre. Comme exemples de sels hydrosolubles plus particulièrement présents dans ledit gel aqueux tel que défini ci-dessus, il y a ceux choisis dans le groupe constitué par le chlorure de sodium, le chlorure de calcium, le chlorure de magnésium, le chlorure d'ammonium, le sulfate de sodium, le sulfate de calcium, le sulfate de magnésium, le sulfate de zinc, le sulfate d'ammonium, le borate de sodium, le glycolate de sodium, le citrate de sodium, le salicylate de sodium, le lactate de sodium, le gluconate de sodium, le gluconate de zinc, le gluconate de manganèse, le gluconate de cuivre, l'aspartate de sodium et l'aspartate de de magnésium.

Selon un autre aspect particulier de la présente invention, le gel aqueux tel que défini précédemment est caractérisé en ce que ledit radical R₁ présent dans les formules (IV) et (V), représente un radical alkyle ramifié R_{1A} de formule CH₃-(CH₂)ₚ-CH(CH₃-(CH₂)₍ₚ₋₂₎)-CH₂-, dans laquelle p représente un nombre entier supérieur ou égal à trois et inférieur ou égal à neuf. Selon cet aspect particulier, ledit alcanol de formule (IV) est de préférence le 2-octyl dodécan-1-ol de formule (IV_{A}) CH₃-(CH₂)₉-CH(CH₃-(CH₂)₇)-CH₂-OH et ladite composition (C) est de préférence une composition (C_{A}) d'alkylpolyxylosides de formules (V_{A}) CH₃-(CH₂)₉-CH(CH₃-(CH₂)₇)-CH₂-O-(G_{A})ₓ-H dans laquelle G_{A}.représente le radical xylosyle ou α,β-D-xylopyranosyle et x représente un nombre décimal compris entre 1,05 et 2,05, plus particulièrement entre 1,10 et 2,00, encore plus particulièrement entre 1,20 et 1,80, ladite composition (C_{A}) consistant, pour 100% molaire, en un mélange de a₁% molaire du composé de formule CH₃-(CH₂)₉-CH(CH₃-(CH₂)₇)-CH₂-O-(G_{A})₁-H, a₂% molaire du composé de formule CH₃-(CH₂)₉-CH(CH₃-(CH₂)₇)-CH₂-O-(G_{A})₂-H, a₃% molaire du composé de formule CH₃-(CH₂)₉-CH(CH₃-(CH₂)₇)-CH₂-O-(G_{A})₃-H, a₄% molaire du composé de formule CH₃-(CH₂)₉-CH(CH₃-(CH₂)₇)-CH₂-O-( G_{A})₄-H, et a₅% molaire du composé de formule CH₃-(CH₂)₉-CH(CH₃-(CH₂)₇)-CH₂-O-(G_{A})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

Dans le cadre de cet aspect préféré, l'invention a tout particulièrement pour objet le gel aqueux tel que défini ci-dessus, comprenant un mélange (M_{A}) consistant en, pour 100% de sa masse de 10,0% massique à 50,0% massique de ladite composition (C_{A}) et de 50,0% massique à 90,0% massique dudit alcanol de formule (IV_{A}), en une proportion telle que le rapport massique (R_{MA}) ayant comme numérateur la masse dudit mélange (M_{A}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0. Comme exemple d'un tel mélange (M_{A}), il y a la composition comprenant pour 100% de sa masse 75,0% massique de 2-octyl dodécanol et 25,0% massique de (2-octyl dodécyl) polyxylosides issus de la réaction en milieu acide du xylose et du 2-octyl dodécanol, commercialisée par la société SEPPIC sous le nom Fluidanov^{™} 20X.

Selon un autre aspect particulier le gel aqueux tel que défini précédemment, est caractérisé en ce que ledit radical R₁ présent dans les formules (IV) et (V), représente un radical alkyle linéaire R_{1B} comportant de douze à vingt-deux atomes de carbone et tout particulièrement de sept à vingt-deux atomes de carbone.

Selon cet aspect particulier, le gel aqueux tel que défini ci-dessus est caractérisé en ce que ledit alcanol de formule (IV) ou ledit mélange d'alcanols de formules (IV), est de préférence un mélange d'alcanols de formules (IV_{B}) R_{1B}-OH dans lesquelles le radical R_{1B} représente un radical alkyle linéaire comportant de vingt à vingt-deux atomes de carbone, et ladite composition (C) est de préférence une composition (C_{A}) d'alkylpolyglucosides de formules (V_{B}) R_{1B}-O-(G_{B})ₓ-H, dans laquelle le radical R_{1B} est tel que défini ci-dessus, G_{B} représente le radical glucosyle ou α, β -D-glucopyranosyle et x représente un nombre décimal compris entre 1,05 et 2,05, plus particulièrement entre 1,10 et 2,00, encore plus particulièrement entre 1,20 et 1,80, ladite composition (C_{B}) consistant, pour 100% molaire, en un mélange de a₁% molaire du composé de formule R_{1B}-O-(G_{B})₁-H, a₂% molaire du composé de formule R_{1B}-O-(G_{B})₂-H, a₃% molaire du composé de formule R_{1B}-O-(G_{B})₃-H, a₄% molaire du composé de formule R_{1B}-O-(Ge)₄-H et as% molaire du composé de formule R_{1B}-O-(G_{B})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x. Dans le cadre de cet aspect préféré, l'invention a plus particulièrement pour objet le gel aqueux tel que défini ci-dessus, caractérisé en ce qu'il comprend un mélange (M_{B}) consistant en, pour 100% de sa masse, de 50,0% massique à 90,0% massique d'un mélange d'alcool arachidylique et d'alcool béhénylique et de 10,0% massique à 50,0% massique d'une composition (C_{B}) d'alkyl polyglucosides issue de la réaction en milieu acide du glucose et dudit mélange d'alcool béhénylique et d'alcool arachidylique, en une proportion telle que le rapport massique (R_{MB}) ayant comme numérateur la masse dudit mélange (M_{B}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0. Comme exemple d'un tel mélange (M_{B}), il y a la composition comprenant pour 100% de sa masse, 80,0% massique d'un mélange équimassique d'alcool béhénylique et d'alcool arachidylique et 20,0% massique d'alkyl polyglucosides issus de la réaction en milieu acide du glucose et dudit mélange équimassique d'alcool béhénylique et d'alcool arachidylique, commercialisée sous le nom Montanov^{™} 202 par la société SEPPIC.

Par huile, on désigne un composé et/ou un mélange de composés insoluble dans l'eau, et liquide à 25 degrés centigrade, et plus particulièrement:
- Les alcanes linéaires comportant de onze à dix-neuf atomes de carbone;
- Les alcanes ramifiés, comportant de sept à quarante atomes de carbone, comme l'isododécane, l'isopentadecane, l'isohexadecane, l'isoheptadecane, l'isooctadecane, l'isononadecane ou l'isoeicosane), ou des mélanges de certains d'entre eux comme ceux cités ci-apres et identifiés par leur nom INCl: C7-8 isoparaffin, Cs-9 isoparaffin, C9-11 isoparaffin, C9-12 isoparaffin, C9-13 isoparaffin, C9-14 isoparaffin, C9-16 isoparaffin, Cio-n isoparaffin, C10-12 isoparaffin, C10-13 isoparaffin, Cn-12 isoparaffin, Cn-13 isoparaffin, Cn-14 isoparaffin, C12-14 isoparaffin, C12-20 isoparaffin, C13-14 isoparaffin, C13-16 isoparaffin;

- Les cyclo-alcanes optionnellement substitues par un ou plusieurs radicaux alkyles linéaires ou ramifiés;
- Les huiles blanches minérales, comme celles commercialisées sous les noms suivants: Marcol^{™}52, Marcol^{™}82, Drakeol^{™}6VR, Eolane^{™}130, Eolane^{™}150;
- L'hemisqualane (ou 2,6,10-trimethyl- dodecane; numero CAS: 3891-98-3), le squalane (ou 2,6,10,15,19,23-hexamethyltetracosane), le polyisobutene hydrogene ou le polydecene hydrogene;
- Les mélanges d'alcanes comportant de 15 à 19 atomes de carbone, lesdits alcanes étant des alcanes linéaires, des alcanes ramifiés et des cyclo-alcanes, et plus particulièrement le mélange (Mi) qui comprend pour 100 percent de sa masse, une proportion massique en alcanes ramifiés superieure ou egale a 90 percent et inferieure ou egale a 100 percent; une proportion massique en alcanes linéaires superieure ou egale a 0 percent et inferieure ou egale a 9 percent, et plus particulièrement inférieure à 5 percent et une proportion massique en cyclo-alcanes superieure ou egale a 0 percent et inferieure ou egale a 1 percent, par exemple les mélanges commercialisés sous les noms Emogreen^{™}L15 ou Emogreen^{™}L19; - Les ethers d'alcool gras de formule (VIII):
   Z1-O-Z2 (VIII), dans laquelle Zi et Z2 identiques ou différents, représentent un radical alkyle linéaire ou ramifié comportant de cinq à dix-huit atomes de carbone, par exemple les dioctyl ether, didecyl ether, didodecyl ether, dodecyl octyl ether, dihexadecyl ether, (1,3-dimethyl butyl) tetradecyl ether, (1,3-dimethyl butyl) hexadecyl ether, le bis(1,3-dimethyl butyl) ether ou le dihexyl ether.
- Les mono-esters d'acides gras et d'alcools de formule (IX):
   R'4-(C=0)-0-R'S (IX), dans laquelle RV(C=0) represente un radical acyle, sature ou insature, lineaire ou ramifie, comportant de huit a vingt-quatre atomes de carbone, et R'5 represente, independamment de R'4, une chaine hydrocarbonee saturee ou insaturee, lineaire ou ramifiee comportant de un a vingt-quatre atomes de carbone, par exemple les laurate de methyle, laurate d'ethyle, laurate de propyle, laurate d'isopropyle, laurate de butyle, laurate de 2-butyle, laurate d'hexyle, cocoate de methyle, cocoate d'ethyle, cocoate de propyle, cocoate d'isopropyle, cocoate de butyle, cocoate de 2-butyle, cocoate d'hexyle, myristate de methyle, myristate d'ethyle, myristate de propyle, le myristate d'isopropyle, le myristate de butyle, le myristate de 2-butyle, le myristate d'hexyle, le myristate d'octyle, le palmitate de methyle, le palmitate d'ethyle, le palmitate de propyle, le palmitate d'isopropyle, le palmitate de butyle, le palmitate de 2-butyle, le palmitate d'hexyle, le palmitate d'octyle, l'oleate de methyle, G oleate d'ethyle, l'oleate de propyle, l'oleate d'isopropyle, l'oleate de butyle, l'oleate de 2-butyle, l'oleate d'hexyle, l'oleate d'octyle, le stearate de methyle, le stearate d'ethyle, le stearate de propyle, le stearate d'isopropyle, le stearate de butyle, le stearate de 2-butyle, le stearate d'hexyle, le stearate d'octyle, l'isostearate de methyle, l'isostearate d'ethyle, l'isostearate de propyle, l'isostearate d'isopropyle, l'isostearate de butyle, l'isostearate de 2-butyle, l'isostearate d'hexyle, l'isostearate d'isostearyle;
- Les di-esters d'acides gras et de glycerol de formule (X) et de formule (XI):

   R'6-(C=0)-0-CH2-CH(0H)-CH2-0-(C=0)-R"7 (X)

   R'8-(C=0)-0-CH2-CH0-(C=0)-R'9-CH2-0H (XI), formules (X) (XI) dans lesquelles R'6(C=O), R'7(C=O), R'8(C=O), R'9-(C=O), identiques ou différents, représentent un groupement acyle, saturé ou insaturé, linéaire ou ramifié, comportant de huit à vingt-quatre atomes de carbone;
- Les tri-esters d'acides gras et de glycérol de formule (XII):
   R'10-(C=O)-O-CH2-CHO-(C=O)-R'11-CH2-O-(C=O)-R'12 (XII), dans laquelle R'10-(C=O), R'11-(C=O) et R'12-(C=O), identiques ou différents, représentent un groupement acyle, saturé ou insaturé, linéaire ou ramifié, comportant de huit à vingt-quatre atomes de carbone.
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes;
- Les huiles végétales éthoxylées. L'invention a aussi pour objet un procédé de soin cosmétique de la peau caractérisé en ce qu'il comprend les étapes successives suivantes :
   une étape de nettoyage de la peau ;
   suivie d'une étape d'application sur la peau nettoyée, du gel aqueux tel que défini précédemment, et
   optionnellement suivie d'une étape d'application sur la peau de la formulation de soin cosmétique souhaitée.

L'invention a également pour objet l'utilisation, soit d'un alcanol de formule (IV) R₁-OH, dans laquelle R₁ représente un radical alkyle linéaire ou ramifié comportant de sept à vingt-deux atomes de carbone, ou un mélange d'alcanols de formule (IV) ; soit d'une composition (C) d'alkyl polyglycosides représentés par les formules (V) R₁-O-(G)ₓ-H, dans lesquelles G représente ou bien un reste du glucose ou bien un reste du xylose et x représente un nombre décimal compris entre 1,05 et 2,05, plus particulièrement entre 1,10 et 2,00, encore plus particulièrement entre 1,20 et 1,80, ladite composition (C) consistant, pour 100% molaire, en un mélange de a₁% molaire du composé de formule R₁-O-(G)₁-H, a₂% molaire du composé de formule R₁-O-(G)₂-H, a₃% molaire du composé de formule R₁-O-(G)₃-H, a₄% molaire du composé de formule R₁-O-(G)₄-H et a₅% molaire du composé de formule R₁-O-(G)₅-H, tel que la somme a₁% + a₂% + a₃% +a₄% + a₅% est égale à 100% molaire et que la somme a₁% + 2a₂% + 3a₃% + 4a₄% + 5a₅% est égale à 100x ; soit d'un mélange (M) dudit alcanol de formule (IV) ou dudit mélange d'alcanols de formules (IV) et de ladite composition (C), ladite utilisation étant caractérisée en ce qu'elle est en vue de réduire ou d'éliminer l'effet collant ressenti lors de l'application sur la peau d'un gel aqueux comprenant pour 100% de sa masse de 0,5% massique à 5,0% en massique un polyélectrolyte anionique réticulé (PA) comportant pour 100% molaire, de 5,0% molaire à 95,0% molaire, d'unités monomériques issues de d'acide 2-méthyl-2-((1-oxo-2-propènyl) amino)-1-propanesulfonique de formule (I) (CH₂=CH-C(=O)-NH)-C(CH₃)₂- S(=O)₂-OH, partiellement ou totalement salifié sous forme de sel alcalin ou de sel d'ammonium, de 4,9% molaire à 90,0% molaire d'unités monomériques issues d'un monomère de formule (II) CH₂=CH-C(=O)-N(R₄)₂ dans laquelle R₄ représente un radical alkyl linéaire ou ramifié comportant de un à quatre atomes de carbone, et de 0,1% molaire à 5,0% molaire d'unités monomériques issues d'un monomère de formule (III) CH₂=C(CH₃)-C(=O)-O-(CH₂-CH₂-O-)ₙ-R₅, dans laquelle R₅ représente un radical alkyle linéaire ou ramifié comportant de huit à vingt atomes de carbone et n représente un nombre supérieur ou égal à 1 et inférieur ou égal à 20.

Par effet collant ressenti lors de l'application d'un gel aqueux sur la peau, on désigne un effet induit par la force d'adhésion dudit gel sur la peau. Dans le cadre de la présente invention, la mesure de la force d'adhésion est réalisée au moyen d'un rhéomètre, notamment le Rhéomètre DHR2^{™} commercialisé par la société Texas Instruments. la force d'adhésion d'un gel doit être inférieure à quatre newtons, pour que l'utilisateur ne ressente pas cet effet collant lors de son l'application sur sa peau.

Selon un aspect particulier, l'utilisation telle que définie précédemment, est caractérisée en ce que ledit polyélectrolyte anionique réticulé (PA) comporte pour 100% molaire de 60,0% molaire à 80,0% molaire d'unités monomérique issues de l'acide de formule (III), partiellement salifié sous forme de sel d'ammonium, de 15,0% molaire à 39,5% molaire d'unités monomériques issues du N,N-diméthyl acrylamide, et de 0,5% molaire à 5,0% molaire d'unités monomériques issues du méthacrylate de lauryle tétra-éthoxylé. Comme exemple d'un tel polyélectrolyte réticulé anionique (PA), il y a le SEPIMAX ZEN^{™} commercialisé par la société SEPPIC, qui un terpolymère réticulé au propanetriacrylate de triméthylol, comprenant pour 100% molaire, 77,4% molaire d'unités monomériques issues du 2-méthyl-2-((1-oxo-2-propényl) amino) 1-propanesulfonate d'ammonium, 19,2% molaire d'unités monomériques issues du N,N-diméthyl acrylamide et 3,4% molaire d'unités monomériques issues du méthacrylate de lauryle tétra-éthoxylé.

Selon un autre aspect particulier, l'utilisation telle que définie précédemment, est caractérisée en ce que ledit radical R₁ présent dans les formules (IV) et (V), représente un radical alkyle ramifié R_{1A} de formule CH₃-(CH₂)ₚ-CH(CH₃-(CH₂)₍ₚ₋₂₎)-CH₂-, dans laquelle p représente un nombre entier supérieur ou égal à trois et inférieur ou égal à neuf. Selon cet aspect particulier, l'utilisation telle que définie ci-dessus est caractérisée en ce que ledit alcanol de formule (IV) est de préférence le 2-octyl dodécan-1-ol de formule (IV_{A}) CH₃-(CH₂)₉-CH(CH₃-(CH₂)₇)-CH₂-OH et ladite composition (C) est de préférence une composition (C_{A}) d'alkylpolyxylosides de formules (V_{A}) CH₃-(CH₂)₉-CH(CH₃-(CH₂)₇)-CH₂-O-(G_{A})ₓ-H dans laquelle G_{A} représente le radical xylosyle ou α, β -D-xylopyranosyle, x représente un nombre décimal compris entre 1,05 et 2,05, plus particulièrement entre 1,10 et 2,00, encore plus particulièrement entre 1,20 et 1,80, ladite composition (C_{A}) consistant, pour 100% molaire, en un mélange de a₁% molaire du composé de formule CH₃-(CH₂)₉-CH(CH₃-(CH₂)₇)-CH₂-O-(G_{A})₁-H, a₂% molaire du composé de formule CH₃-(CH₂)₉-CH(CH₃-(CH₂)₇)-CH₂-O-(G_{A})₂-H, a₃% molaire du composé de formule CH₃-(CH₂)₉-CH(CH₃-(CH₂)₇)-CH₂-O-(G_{A})₃-H, a₄% molaire du composé de formule CH₃-(CH₂)₉-CH(CH₃-(CH₂)₇)-CH₂-O-( G_{A})₄-H, et a₅% molaire du composé de formule CH₃-(CH₂)₉-CH(CH₃-(CH₂)₇)-CH₂-O-(G_{A})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x. Dans le cadre de cet aspect préféré, l'invention a plus particulièrement pour objet, l'utilisation telle que définie ci-dessus, d'un mélange (M_{A}) consistant en, pour 100% de sa masse, de 10,0% massique à 50,0% massique de ladite composition (C_{A}) et de 50,0% massique à 90,0% massique dudit alcanol de formule (IV_{A}). Comme exemple d'un tel mélange (M_{A}), il y a la composition comprenant pour 100% de sa masse, 75,0% massique de 2-octyl dodécanol et 25,0% massique de (2-octyl dodécyl) polyxylosides, commercialisée par la société SEPPIC sous le nom Fluidanov^{™} 20X.

Selon un autre aspect particulier, l'utilisation telle que définie précédemment est caractérisée en ce que ledit radical ledit radical R₁ présent dans les formules (IV) et (V), représente un radical alkyle linéaire R_{1B} comportant de sept à vingt-deux atomes de carbone et tout particulièrement de sept à vingt atomes de carbone. Selon cet aspect particulier, l'utilisation telle que définie ci-dessus est caractérisée en ce que ledit alcanol de formule (IV) ou ledit mélange d'alcanols de formules (IV), est de préférence un mélange d'alcanols de formules (IV_{B}) R_{1B}-OH dans lesquelles le radical R_{1B} représente un radical alkyle linéaire comportant de vingt à vingt-deux atomes de carbone, et ladite composition (C) est de préférence une composition (C_{B}) d'alkylpolyglucosides de formules (V_{B}) R_{1B}-O-(G_{B})ₓ-H, dans lesquelles le radical R_{1B} est tel que défini ci-dessus, G_{B} représente le radical glucosyle ou α,β-D-glucopyranosye et x représente un nombre décimal compris entre 1,05 et 2,05, plus particulièrement entre 1,10 et 2,00, encore plus particulièrement entre 1,20 et 1,80, ladite composition (C_{B}) consistant, pour 100% molaire, en un mélange de a₁% molaire du composé de formule R_{1B}-O-(Ga)₁-H, a₂% molaire du composé de formule R_{1B}-O-(G_{B})₂-H, a₃% molaire du composé de formule R_{1B}-O-(G_{B})₃-H, a₄% molaire du composé de formule R_{1B}-O-(G_{B})₄-H et a₅% molaire du composé de formule R_{1B}-O-(G_{B})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x. Dans le cadre de cet aspect préféré, l'invention a plus particulièrement pour objet l'utilisation telle que définie ci-dessus, d'un mélange (M_{B}) consistant en, pour 100% de sa masse, de 50,0% massique à 90,0% massique d'un mélange d'alcool arachidylique et d'alcool béhénylique) et de 10,0% massique à 50,0% massique d'une composition (C_{B}) d'alkyl polyglucosides issue de la réaction en milieu acide du glucose et dudit mélange d'alcool béhénylique et d'alcool arachidylique. Comme exemple d'un tel mélange (M_{B}) tel que défini ci-dessus, il y a la composition comprenant pour 100% de sa masse, 80,0% massique d'un mélange équimassique d'alcool béhénylique et d'alcool arachidylique et 20,0% massique d'alkyl polyglucosides issus de la réaction en milieu acide du glucose et dudit mélange équimassique d'alcool béhénylique et d'alcool arachidylique, commercialisée sous le nom Montanov^{™} 202 par la société SEPPIC.

L'invention a encore pour objet un procédé pour réduire ou éliminer l'effet collant ressenti lors de l'application sur la peau d'un gel aqueux comprenant pour 100% de sa masse, de 0,5% massique à 5,0% massique d'un polyélectrolyte anionique réticulé (PA) comportant pour 100% molaire de 5,0% molaire à 95,0% molaire, d'unités monomériques issues de d'acide 2-méthyl-2-((1-oxo-2-propènyl) amino)-1-propanesulfonique de formule (I) (CH₂=CH-C(=O)-NH)-C(CH₃)₂-S(=O)₂-OH, partiellement ou totalement salifié sous forme de sel alcalin ou de sel d'ammonium, de 4,9% molaire à 90,0% molaire d'unités monomériques issues d'un monomère de formule (II) CH₂=CH-C(=O)-N(R₄)₂ dans laquelle R₄ représente un radical alkyl linéaire ou ramifié comportant de un à quatre atomes de carbone, et de 0,1% molaire à 5,0% molaire d'unités monomériques issues d'un monomère de formule (III) CH₂=C(CH₃)-C(=O)-O-(CH₂-CH₂-O-)ₙ-R₅, dans laquelle R₅ représente un radical alkyle linéaire ou ramifié comportant de huit à vingt atomes de carbone et n représente un nombre supérieur ou égal à 1 et inférieur ou égal à 20, caractérisé en ce que l'on modifie la composition de ce gel aqueux, avant son application sur la peau, en y ajoutant, soit un alcanol de formule (IV) R₁-OH, dans laquelle R₁ représente un radical alkyle linéaire ou ramifié comportant de huit à vingt-deux atomes de carbone, ou un mélange d'alcanols de formules (IV) ; soit une composition (C) d'alkyl polyglycosides représentés par les formules (V) R₁-O-(G)ₓ-H, dans lesquelles G représente ou bien le reste du glucose ou bien le reste du xylose et x représente un nombre décimal compris entre 1,05 et 2,05, plus particulièrement entre 1,10 et 2,00, encore plus particulièrement entre 1,20 et 1,80, ladite composition (C) consistant, pour 100% molaire, en un mélange de a₁% molaire du composé de formule R₁-O-(G)₁-H, a₂% molaire du composé de formule R₁-O-(G)₂-H, a₃% molaire du composé de formule R₁-O-(G)₃-H, a₄% molaire du composé de formule R₁-O-(G)₄-H et a₅% molaire du composé de formule R₁-O-(G)₅-H, tel que la somme a₁% + a₂% + a₃% +a₄% + a₅% est égale à 100% molaire et que la somme a₁% + 2a₂% + 3a₃% + 4a₄% + 5a₅% est égale à 100x ; soit un mélange (M) dudit alcanol de formule (IV) ou dudit mélange d'alcanols de formules (IV) et de ladite composition (C), en une proportion telle que le rapport massique (R_{M}) au sein du gel aqueux ainsi modifié, ayant comme numérateur ou bien la masse dudit alcanol de formule (IV) ou dudit mélange d'alcanols de formules (IV), ou bien la masse de ladite composition (C), ou bien la masse dudit mélange (M) et comme dénominateur, la masse dudit polyélectrolyte anionique réticulé (PA), est supérieur ou égal à 1,0 et inférieur ou égal à 10,0 et plus particulièrement, supérieur ou égal à 1,5 et inférieur ou égal à 5,0.

Par effet collant ressenti lors de l'application d'un gel aqueux sur la peau, on désigne un effet induit par la force d'adhésion dudit gel sur la peau. Dans le cadre de la présente invention, la mesure de la force d'adhésion est réalisée au moyen d'un rhéomètre, notamment le Rhéomètre DHR2^{™} commercialisé par la société Texas Instruments. la force d'adhésion d'un gel doit être inférieure à quatre newtons, pour que l'utilisateur ne ressente pas cet effet collant lors de son l'application sur sa peau.

Selon un aspect particulier, le procédé tel que défini ci-dessus est caractérisé en ce que ledit polyélectrolyte anionique réticulé (PA) comporte pour 100% molaire, de 60,0% molaire à 80,0% molaire d'unités monomérique issues de l'acide de formule (III), partiellement salifié sous forme de sel d'ammonium, de 15,0% molaire à 39,5% molaire d'unités monomériques issues du N,N-diméthyl acrylamide, et de 0,5% molaire à 5,0% molaire d'unités monomériques issues du méthacrylate de lauryle tétra-éthoxylé.

Selon un autre aspect particulier, le procédé tel que défini ci-dessus est caractérisé en ce que ledit radical R₁ présent dans les formules (IV) et (V), représente un radical alkyle ramifié R_{1A} de formule CH₃-(CH₂)ₚ-CH(CH₃-(CH₂)₍ₚ₋₂₎)-CH₂-, dans laquelle p représente un nombre entier supérieur ou égal à trois et inférieur ou égal à neuf. Selon cet aspect particulier, le procédé tel que défini ci-dessus est caractérisé en ce que ledit alcanol de formule (IV) est de préférence le 2-octyl dodécan-1-ol de formule (I_{A}) CH₃-(CH₂)₉-CH(CH₃-(CH₂)₇)-CH₂-OH et ladite composition (C) est de préférence une composition (C_{A}) d'alkylpolyxylosides de formules (V_{A}) CH₃-(CH₂)₉-CH(CH₃-(CH₂)₇)-CH₂-O-(G_{A})ₓ-H dans laquelle G_{A}.représente le radical xylosyle ou α,β-D-xylopyranosyle, x représente un nombre décimal compris entre 1,05 et 2,05, plus particulièrement entre 1,10 et 2,00, encore plus particulièrement entre 1,20 et 1,80, ladite composition (C_{A}) consistant, pour 100% molaire, en un mélange de a₁% molaire du composé de formule CH₃-(CH₂)₉-CH(CH₃-(CH₂)₇)-CH₂-O-(G_{A})₁-H, a₂% molaire du composé de formule CH₃-(CH₂)₉-CH(CH₃-(CH₂)₇)-CH₂-O-(G_{A})₂-H, a₃% molaire du composé de formule CH₃-(CH₂)₉-CH(CH₃-(CH₂)₇)-CH₂-O-(G_{A})₃-H, a₄% molaire du composé de formule CH₃-(CH₂)₉-CH(CH₃-(CH₂)₇)-CH₂-O-( G_{A})₄-H, et a₅% molaire du composé de formule CH₃-(CH₂)₉-CH(CH₃-(CH₂)₇)-CH₂-O-(G_{A})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x. Dans le cadre de cet aspect préféré, l'invention a plus particulièrement pour objet le procédé tel défini ci-dessus, caractérisé en ce que l'on modifie la composition dudit gel aqueux avant son application sur la peau, en y ajoutant un mélange (M_{A}) consistant en, pour 100% de sa masse, de 10,0% massique à 50,0% massique de ladite composition (C_{A}) et de 50,0% massique à 90,0% massique dudit alcanol de formule (IV_{A}), en une proportion telle qu'au sein dudit gel aqueux ainsi modifié, le rapport massique (R_{MA}) ayant comme numérateur la masse dudit mélange (M_{A}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0. Comme exemple d'un tel mélange (M_{A}) mis en œuvre dans le procédé tel que défini ci-dessus, il y a la composition comprenant pour 100% de sa masse, 75,0% massique de 2-octyl dodécanol et 25,0% massique de (2-octyl dodécyl) polyxylosides, commercialisée par la société SEPPIC sous le nom Fluidanov^{™} 20X.

Selon un autre aspect particulier, le procédé tel que défini précédemment est caractérisé en ce que ledit radical R₁ présent dans les formules (IV) et (V), représente un radical alkyle linéaire R_{1B} comportant de sept à vingt-deux atomes de carbone et tout particulièrement de douze à vingt-deux atomes de carbone. Selon cet aspect particulier, ledit alcanol de formule (IV) ou ledit mélange d'alcanols de formules (IV), est de préférence un mélange d'alcanols de formules (IV_{B}) R_{1B}-OH dans lesquelles le radical R_{1B} représente un radical alkyle linéaire comportant de vingt à vingt-deux atomes de carbone et ladite composition (C) est de préférence une composition (C_{B}) d'alkylpolyglucosides de formules (V_{B}) R_{1B}-O-(G_{B})ₓ-H, dans lesquelles le radical R_{1B} est tel que défini ci-dessus, G_{B} représente le radical glucosyle ou α,β-D-glucopyranosyle et x représente un nombre décimal compris entre 1,05 et 2,05, plus particulièrement entre 1,10 et 2,00, encore plus particulièrement entre 1,20 et 1,80, ladite composition (C_{B}) consistant pour 100% molaire, en un mélange de a₁% molaire du composé de formule R_{1B}-O-(G_{B})₁-H, a₂% molaire du composé de formule R_{1B}-O-(G_{B})₂-H, a₃% molaire du composé de formule R_{1B}-O-(G_{B})₃-H, a₄% molaire du composé de formule R_{1B}-O-(G_{B})₄-H, et a₅% molaire du composé de formule R_{1B}-O-(G_{B})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x. Dans le cadre de cet aspect préféré, l'invention a plus particulièrement pour objet le procédé tel défini ci-dessus, caractérisé en ce que l'on modifie, avant son application sur la peau, la composition dudit gel aqueux, avant son application sur la peau, en y ajoutant un mélange d'un mélange (M_{B}) consistant en, pour 100% de sa masse, de 50,0% massique à 90,0% massique d'un mélange d'alcool arachidylique et d'alcool béhénylique et de 10,0% massique à 50,0% massique d'une composition (C_{B}) d'alkyl polyglucosides issue de la réaction en milieu acide du glucose et dudit mélange d'alcool béhénylique et d'alcool arachidylique, en une proportion telle qu'au sein dudit gel aqueux ainsi modifié, le rapport massique (R_{MB}) ayant comme numérateur la masse dudit mélange (M_{B}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0. Comme exemple d'un tel mélange (M_{B}) mis en œuvre dans le procédé tel que défini ci-dessus, il y a la composition comprenant pour 100% de sa masse, 80,0% massique d'un mélange équimassique d'alcool béhénylique et d'alcool arachidylique et 20,0% massique d'alkyl polyglucosides issus de la réaction en milieu acide du glucose et dudit mélange équimassique d'alcool béhénylique et d'alcool arachidylique, commercialisée sous le nom Montanov^{™} 202 par la société SEPPIC.

La partie expérimentale suivante illustre l'invention sans toutefois la modifier.

### Préparation de gels aqueux.

Les gels aqueux A, A', B, C, E, E', F, G, H, L, M, N, P selon l'invention et les gels témoins T1, T'1 et T2, sont préparés par mélange de leurs constituants. Leurs compositions respectives sont consignées dans les tableaux 1 à 5 suivants, accompagnées dans les tableaux 4 et 5 de celles des solutions témoins T1, T'1 et T2. Le procédé pour leur préparation commun à tous est le suivant :
- On verse dans un réacteur muni d'une agitation mécanique et d'une double enveloppe comprenant un fluide caloporteur, à une température de 20°C, la quantité nécessaire d'eau qui est ensuite amenée à une température de 25°C sous agitation mécanique de type Rayneri munie d'une défloculeuse à une vitesse de mille tours par minute ;
- le polyélectrolyte anionique réticulé (PA1) est ensuite ajouté progressivement à cette température de 25°C sous agitation mécanique ;

- - après homogénéisation du gel obtenu précédemment, l'agent anti-collant à tester est ensuite ajouté progressivement à la température de 25°C sous agitation mécanique à la vitesse de mille tours par minute ;
- - le mélange obtenu est ensuite maintenu à une température de 25°C, puis, si nécessaire, le sel est ajouté dans le milieu précédemment obtenu, toujours sous agitation mécanique.

Les gels D, D',I et J sont préparés comme ci-dessus mais une température comprise entre 75°C et 85°C. L'ajout éventuel de sel est effectué après refroidissement à 40°C.

**Tableau 1**

| | Composition des gels aqueux (% massiques) | | | |
|---|---|---|---|---|
| Composants ↓ | A | B | C | D |
| Polyélectrolyte anionique réticulé PA₁ | 1,0% | 1,0% | 1,0% | 1,0% |
| Fluidanov^{™} 20X | 2,0% | 3,0% | 5,0% | 0,0% |
| Alcool arachidylique | 0,0% | 0,0% | 0,0% | 5,0% |
| 2-octyl dodécanol | 0,0% | 0,0% | 0,0% | 0,0% |
| Eau | qsp 100% | qsp 100% | qsp 100% | qsp 100% |
| NaCl | 0% | 0% | 0% | 0% |

**Tableau 2**

| | Composition des gels aqueux (% massiques) | | | |
|---|---|---|---|---|
| Composants ↓ | E | A' | D' | E' |
| Polyélectrolyte anionique réticulé PA₁ | 1,0% | 1,0% | 1,0% | 1,0% |
| Fluidanov^{™} 20X | 0,0% | 2,0% | 0,0% | 0,0% |
| Alcool arachidylique | 0,0% | 0,0% | 2,0% | 0,0% |
| 2-octyl dodécanol | 5,0% | 0,0% | 0,0% | 2,0% |
| Eau | qsp 100% | 1,0% | 1,0% | 1,0% |

**Tableau 3**

| | Composition des gels aqueux (% massiques) | | |
|---|---|---|---|
| Composants ↓ | F | G | H |
| Polyélectrolyte anionique | 1,0% | 1,0% | 1,0% |
| réticulé PA₁ | | | |
| Fluidanox^{™}20X | 2,0% | 0,5% | 1,0% |
| Alcool arachidylique | 0,0% | 0,0% | 0,0% |
| 2-octyl dodécanol | 0,0% | 0,0% | 0,0% |
| NaCl | 1,0% | 1,0% | 1,0% |
| Eau | qsp 100% | qsp 100% | qsp 100% |

**Tableau 4**

| | Composition des gels aqueux (% massiques) | | | |
|---|---|---|---|---|
| Composants ↓ | I | J | T₁ | T₂ |
| Polyélectrolyte anionique réticulé PA₁ | 1,0% | 1,0% | 1,0% | 1,0% |
| Fluidanov^{™} 20X | 0,0% | 0,0% | 0,0% | 0,0% |
| Alcool myristylique | 2,0% | 0,0% | 0,0% | 0,0% |
| Montanov^{™} 202 | 0,0% | 2,0% | 0,0% | 0,0% |
| NaCl | 1,0% | 1,0% | 0,0% | 1,0% |
| Eau | qsp 100% | qsp 100% | qsp 100% | qsp 100% |

**Tableau 5**

| | Composition des gels aqueux (% massiques) ↓ | | | | |
|---|---|---|---|---|---|
| Composants ↓ | L | M | N | P | T'₁ |
| Polyélectrolyte anionique réticulé PA₁ | 1,0% | 1,0% | 1,0% | 1,0% | 1,0% |
| Octanol-1/Décanol-1 (50/50 massique) | 2,0% | 0,0% | 0,0% | 0,0% | 0,0% |
| Oramix^{™}CG 110 | 0,0% | 2,0% | 0,0% | 0,0% | 0,0% |
| Heptanol-1 | 0,0% | 0,0% | 2,0% | 0,0% | 0,0% |
| Sepiclear^{™} G7 | 0,0% | 0,0% | 0,0% | 2,0% | 0,0% |
| NaCl | 1,0% | 1,0% | 1,0% | 1,0% | 1,0% |
| Eau | qsp 100% | qsp 100% | qsp 100% | qsp 100% | qsp 100% |

- Le polyélectrolyte anionique réticulé PA₁ est un terpolymère réticulé au propanetriacrylate de triméthylol, comprenant pour 100% molaire, 77,4% molaire d'unités monomériques issues du 2-méthyl-2-((1-oxo-2-propényl) amino) 1-propanesulfonate d'ammonium, 19,2% molaire d'unités monomériques issues du N,N-diméthyl acrylamide et 3,4% molaire d'unités monomériques issues du méthacrylate de lauryle tétra-éthoxylé ;
- Le Fluidanov^{™} 20X est une composition comprenant pour 100% de sa masse 75,0% massique de 2-octyl dodécanol et 25,0% massique de (2-octyl dodécyl) polyxylosides issus de la réaction en milieu acide du xylose et du 2-octyl dodécanol ;
- Le Montanov^{™} 202 est une composition comprenant pour 100% de sa masse, 80,0% massique d'un mélange équimassique d'alcool béhénylique et d'alcool arachidylique et 20,0% massique d'alkyl polyglucosides issus de la réaction en milieu acide du glucose et dudit mélange équimassique d'alcool béhénylique et d'alcool arachidylique.
- Oramix^{™} CG 110 est une composition comprenant pour 100% de sa masse 99,0% massique d'un mélange 50/50 massique de 1-octyl polyglucosides et de 1-décyl polyglucosides et 1,0% massique d'eau issus de la réaction en milieu acide du glucose et d'un mélange 50/50 massique de 1-octanol et de 1-décanol ;
- Sepiclear^{™}G7 est une composition comprenant pour 100% de sa masse 99,0% massique de n-heptanyl polyglucosides et de 1,0% massique de n-heptanol-1

### Analyse des gels aqueux

- L'aspect des gels aqueux est apprécié visuellement après sept jours de stockage à 25°C et à 45°C ;
- Les viscosités (en mPa.s) des gels aqueux sont mesurées au moyen d'un viscosimètre Brookfield^{™} RVT muni du mobile 3 à une vitesse de de rotation de six tours par minute. Les viscosités acceptables sont comprises entre 5.000mPas. et 50.000mPa.s.
- L'effet collant est déterminé au moyen d'un Rhéomètre DHR2^{™} de la société Texas Instruments, par mesure de la force d'adhésion d'un échantillon de cent microlitres de gel aqueux sur une plaque de PMMA disposée sur un plan Peltier, lors d'une contrainte rotationnelle du plan mobile supérieur sur le gel, pendant dix secondes. La force normale (en Newton) qui retient le plan mobile supérieur au plan Peltier (ou force d'adhésion) est mesurée lors d'une succession de dix mouvements de compression suivis de remontées. On procède à cinq mesures par gel. La force d'adhésion du gel doit être inférieure à quatre Newton, pour que l'utilisateur ne ressente pas cet effet collant lors son l'application sur sa peau. Les résultats des analyses sont consignés dans les tableaux 6 et 7 suivants :

**Tableau 6**

| | Gels aqueux ↓ | | | | |
|---|---|---|---|---|---|
| | A' | E' | D' | I | J |
| Aspect à 7 jours à 25°C | Homogène | | | | |
| Aspect à 7 jours à 45°C | Homogène | | | | |
| Analyses ↓ | | | | | |
| Viscosité (mPa.s) | 13.200 | 5.300 | 4.300 | 10.800 | 11.400 |
| Force d'adhésion (N) | 3,78 | 3,60 | 3,92 | 3.48 | 3,73 |

**Tableau 7**

| | Gels aqueux ↓ | | | | |
|---|---|---|---|---|---|
| | F | G | H | T₁ | T₂ |
| Aspect à 7 jours à 25°C | Homogène | | | | |
| Aspect à 7 jours à 45°C | Homogène | | | | |
| Analyse ↓ | | | | | |
| Viscosité (mPa.s) | 5.300 | 5.200 | 5.000 | 11.200 | 4.200 |
| Force d'adhésion (N) | 3,37 | 3,26 | 3,06 | 4,47 | 4,24 |

**Tableau 8**

| | Gels aqueux↓ | | | | |
|---|---|---|---|---|---|
| | L | M | N | P | T'₁ |
| Aspect à 7 jours à 25°C | Homogène | Homogène | Homogène | Homogène | Homogène |
| Aspect à 7 jours à 45°C | Homogène | Homogène | Homogène | Homogène | Homogène |
| Analyses ↓ | | | | | |
| Viscosité (mPa.s) | 7.200 | 1.600 | 6.600 | 3.500 | 6.700 |
| Force d'adhésion (N) | 3,43 | 3,21 | 3,17 | 3,18 | 4,58 |

Ces résultats mettent évidence la diminution de l'effet collant induit par la présence au sein d'un gel aqueux épaissi par un polyélectrolyte anionique réticulé, d'un alcool gras ou d'un mélange d'alcools gras comportant de sept à vingt-deux atomes de carbone ou une composition d'alcools gras et d'alkylpolyglycosides dans le rapport massique (R_{M}) tel que revendiqué.

## Revendications

1. Gel aqueux sans huile **caractérisé en ce qu'**il comprend, pour 100% de sa masse :
- de 0,5% massique à 5,0% massique d'un polyélectrolyte anionique réticulé (PA) comportant pour 100% molaire :
• de 5,0% molaire à 95,0% molaire, d'unités monomériques issues de de l'acide 2-méthyl-2-((1-oxo-2-propènyl) amino)-1-propanesulfonique de formule (I) (CH₂=CH-C(=O)-NH)-C(CH₃)₂-S(=O)₂-OH, partiellement ou totalement salifié sous forme de sel alcalin ou de sel d'ammonium,
• de 4,9% molaire à 90,0% molaire d'unités monomériques issues d'un monomère de formule (II) CH₂=CH-C(=O)-N(R₄)₂, dans laquelle R₄ représente un radical alkyl linéaire ou ramifié comportant de un à quatre atomes de carbone, et
• de 0,1% molaire à 5,0% molaire d'unités monomériques issues d'un monomère de formule (III) CH₂=C(CH₃)-C(=O)-O-(CH₂-CH₂-O-)ₙ-R₅, dans laquelle R₅ représente un radical alkyle linéaire ou ramifié comportant de huit à vingt atomes de carbone et n représente un nombre supérieur ou égal à 1 et inférieur ou égal à 20,
- de 0,5% massique à 9,5% massique :
• soit d'un alcanol de formule (IV) R₁-OH, dans laquelle R₁ représente un radical alkyle linéaire ou ramifié comportant de sept à vingt-deux atomes de carbone, ou un mélange d'alcanols de formules (IV) ;
• soit d'une composition (C) d'alkyl polyglycosides représentés par les formules (V) R₁-O-(G)ₓ-H, dans lesquelles G représente ou bien un reste du glucose ou bien un reste du xylose et x représente un nombre décimal compris entre 1,05 et 2,05, ladite composition (C) consistant, pour 100% molaire, en un mélange de a₁% molaire du composé de formule R₁-O-(G)₁-H, a₂% molaire du composé de formule
R₁-O-(G)₂-H, a₃% molaire du composé de formule R₁-O-(G)₃-H, a₄% molaire du composé de formule R₁-O-(G)₄-H, et a₅% molaire du composé de formule R₁-O-(G)₅-H, tel que la somme a₁% + a₂% + a₃% +a₄% + a₅% est égale à 100% molaire et que la somme a₁% + 2a₂% + 3a₃% + 4a₄% + 5a₅% est égale à 100x ;
• soit d'un mélange (M) dudit alcanol de formule (IV) ou dudit mélange d'alcanols de formules (IV) et de ladite composition (C),
- de 0,0% massique à 3,0% massique un ou plusieurs actifs cosmétiques choisis dans le groupe constitué par le rétinol, la vitamine C, le niacinamide (vitamine B3), l'undécylénoyl phénylalanine, l'acide glycolique, l'acide azélaïque, l'acide hyaluronique, le mélange de xylitol, de polyglucosides et de 1,4-anhydro xylitol et l'urée, et
- la quantité nécessaire en eau pour compléter à 100% massique, et **en ce qu'**au sein dudit gel aqueux, le rapport massique (R_{M}) ayant comme numérateur ou bien la masse dudit alcanol de formule (IV) ou dudit mélange d'alcanols de formules (IV) ou bien la masse de ladite composition (C) ou bien la masse dudit mélange (M) et comme dénominateur, la masse dudit polyélectrolyte anionique réticulé (PA), est supérieur ou égal à 1,0 et inférieur ou égal à 10,0.

2. Gel aqueux selon la revendication 1, **caractérisé en ce que** ledit polyélectrolyte anionique réticulé (PA) comporte, pour 100% molaire :
- de 60,0% molaire à 80,0% molaire d'unités monomérique issues de l'acide de formule (III), partiellement salifié sous forme de sel d'ammonium,
- de 15,0% molaire à 39,5% molaire d'unités monomériques issues du N,N-diméthyl acrylamide, et
- de 0,5% molaire à 5,0% molaire d'unités monomériques issues du méthacrylate de lauryle tétra-éthoxylé.

3. Gel aqueux selon l'une quelconque des revendication 1 ou 2, **caractérisé en ce que** dans l'eau constitutive dudit gel aqueux, est dissout au moins un sel hydrosoluble choisi dans le groupe constitué par les sels de métaux alcalins, les sels de métaux alcalino-terreux et les sels de métaux de transition, de sorte que la concentration totale au sein de l'eau en ledit au moins un sel hydrosoluble, est supérieure à 0,00 mole par litre et inférieure ou égale à 1,00 mole par litre.

4. Gel aqueux selon l'une quelconque des revendication 1 à 3, **caractérisé en ce que** ledit alcanol de formule (IV) est le 2-octyl dodécan-1-ol de formule (IV_{A}) CH₃-(CH₂)₉-CH(CH₃-(CH₂)₇)-CH₂-OH et ladite composition (C) est une composition (C_{A}) d'alkylpolyxylosides de formules (V_{A}) CH₃-(CH₂)₉-CH(CH₃-(CH₂)₇)-CH₂-O-(G_{A})ₓ-H dans laquelle G_{A}.représente le radical xylosyle ou α,β-D-xylopyranosyle et x représente un nombre décimal compris entre 1,05 et 2,05, ladite composition (C_{A}) consistant, pour 100% molaire, en un mélange de a₁% molaire du composé de formule CH₃-(CH₂)₉-CH(CH₃-(CH₂)₇)-CH₂-O-(G_{A})₁-H, a₂% molaire du composé de formule CH₃-(CH₂)₉-CH(CH₃-(CH₂)₇)-CH₂-O-(G_{A})₂-H, a₃% molaire du composé de formule CH₃-(CH₂)₉-CH(CH₃-(CH₂)₇)-CH₂-O-(G_{A})₃-H, a₄% molaire du composé de formule CH₃-(CH₂)₉-CH(CH₃-(CH₂)₇)-CH₂-O-( G_{A})₄-H, et a₅% molaire du composé de formule CH₃-(CH₂)₉-CH(CH₃-(CH₂)₇)-CH₂-O-(G_{A})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

5. Gel aqueux selon la revendication 4 **caractérisé en ce qu'**il comprend un mélange (M_{A}) consistant en, pour 100% de sa masse, de 10,0% massique à 50,0% massique de ladite composition (C_{A}) et de 50,0% massique à 90,0% massique dudit alcanol de formule (IV_{A}), en une proportion telle que le rapport massique (R_{MA}) ayant comme numérateur la masse dudit mélange (M_{A}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0.

6. Gel aqueux selon l'une quelconque des revendication 1 à 3, **caractérisé en ce que** ledit alcanol de formule (IV) ou ledit mélange d'alcanols de formule (IV), est un mélange d'alcanols de formules (IV_{B}) R_{1B}-OH dans lesquelles le radical R_{1B} représente un radical alkyle linéaire comportant de vingt à vingt-deux atomes de carbone, et ladite composition (C) est une composition (C_{B}) d'alkylpolyglucosides de formules (V_{B}) R_{1B}-O-(G_{B})ₓ-H, dans laquelle le radical R_{1B} est tel que défini ci-dessus, G_{B} représente le radical glucosyle ou α,β -D-glucopyranosyle et x représente un nombre décimal compris entre 1,05 et 2,05, ladite composition (C_{B}) consistant, pour 100% molaire, en un mélange de a₁% molaire du composé de formule R_{1B}-O-(G_{B})₁-H, a₂% molaire du composé de formule R_{1B}-O-(G_{B})₂-H, a₃% molaire du composé de formule R_{1B}-O-(G_{B})₃-H, a₄% molaire du composé de formule R_{1B}-O-(G_{B})₄-H, et a₅% molaire du composé de formule R_{1B}-O-(G_{B})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

7. Gel aqueux selon la revendication 6, **caractérisé en ce qu'**il comprend un mélange (M_{B}) consistant, pour 100% de sa masse, en de 50,0% massique à 90,0% massique d'un mélange d'alcool arachidylique et d'alcool béhénylique et de 10,0% massique à 50,0% massique d'une composition (C_{B}) d'alkyl polyglucosides issue de la réaction en milieu acide du glucose et dudit mélange d'alcool béhénylique et d'alcool arachidylique, en une proportion telle que le rapport massique (R_{MB}) ayant comme numérateur la masse dudit mélange (M_{B}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0.

8. Procédé de soin cosmétique de la peau **caractérisé en ce qu'**il comprend les étapes successives suivantes :
- une étape de nettoyage de la peau ;
- suivie d'une étape d'application sur la peau nettoyée, du gel aqueux tel que défini à l'une quelconque des revendications 1 à 7, et
- - optionnellement suivie d'une étape d'application sur la peau de la formulation de soin cosmétique souhaitée.

9. Utilisation, soit d'un alcanol de formule (IV) R₁-OH, dans laquelle R₁ représente un radical alkyle linéaire ou ramifié comportant de sept à vingt-deux atomes de carbone, ou un mélange d'alcanols de formules (IV) ; soit d'une composition (C) d'alkyl polyglycosides représentés par les formules (V) R₁-O-(G)ₓ-H, dans lesquelles G représente ou bien un reste du glucose ou bien un reste du xylose et x représente un nombre décimal compris entre 1,05 et 2,05, ladite composition (C) consistant, pour 100% molaire, en un mélange de a₁% molaire du composé de formule R₁-O-(G)₁-H, a₂% molaire du composé de formule R₁-O-(G)₂-H, a₃% molaire du composé de formule R₁-O-(G)₃-H, a₄% molaire du composé de formule (II)₄ R₁-O-(G)₄-H, et a₅% molaire du composé de formule R₁-O-(G)₅-H, tel que la somme a₁% + a₂% + a₃% +a₄% + a₅% est égale à 100% molaire et que la somme a₁% + 2a₂% + 3a₃% + 4a₄% + 5a₅% est égale à 100x ; soit d'un mélange (M) dudit alcanol de formule (IV) ou dudit mélange d'alcanols de formule (IV) et de ladite composition (C), ladite utilisation étant **caractérisée en ce qu'**elle est en vue de réduire ou d'éliminer l'effet collant ressenti lors de l'application sur la peau d'un gel aqueux comprenant pour 100% de sa masse de 0,5% massique à 5,0% en massique un polyélectrolyte anionique réticulé (PA) comportant pour 100% molaire, de 5,0% molaire à 95,0% molaire, d'unités monomériques issues de l'acide 2-méthyl-2-((1-oxo-2-propènyl) amino)-1-propanesulfonique de formule (I) (CH₂=CH-C(=O)-NH)-C(CH₃)₂- S(=O)₂-OH, partiellement ou totalement salifié sous forme de sel alcalin ou de sel d'ammonium ; de 4,9% molaire à 90,0% molaire d'unités monomériques issues d'un monomère de formule (II) CH₂=CH-C(=O)-N(R₄)₂ dans laquelle R₄ représente un radical alkyl linéaire ou ramifié comportant de un à quatre atomes de carbone ; et de 0,1% molaire à 5,0% molaire d'unités monomériques issues d'un monomère de formule (III) CH₂=C(CH₃)-C(=O)-O-(CH₂-CH₂-O-)ₙ-R₅, dans laquelle R₅ représente un radical alkyle linéaire ou ramifié comportant de huit à vingt atomes de carbone et n représente un nombre supérieur ou égal à 1 et inférieur ou égal à 20.

10. Utilisation selon la revendication 9, **caractérisée en ce que** ledit polyélectrolyte anionique réticulé (PA) comporte pour 100% molaire de 60,0% molaire à 80,0% molaire d'unités monomérique issues de l'acide de formule (I), partiellement salifié sous forme de sel d'ammonium, de 15,0% molaire à 39,5% molaire d'unités monomériques issues du N,N-diméthyl acrylamide, et de 0,5% molaire à 5,0% molaire d'unités monomériques issues du méthacrylate de lauryle tétra-éthoxylé.

11. Utilisation selon l'une quelconque des revendications 9 ou 10, **caractérisée en ce que** ledit alcanol de formule (IV) est le 2-octyl dodécan-1-ol de formule (IV_{A}) CH₃-(CH₂)₉-CH(CH₃-(CH₂)₇)-CH₂-OH et ladite composition (C) est une composition (C_{A}) d'alkylpolyxylosides de formules (V_{A}) CH₃-(CH₂)₉-CH(CH₃-(CH₂)₇)-CH₂-O-(G_{A})ₓ-H dans laquelle G_{A}.représente le radical xylosyle ou α,β-D-xylopyranosyle et x représente un nombre décimal compris entre 1,05 et 2,05, ladite composition (C_{A}) consistant, pour 100% molaire, en un mélange de a₁% molaire du composé de formule CH₃-(CH₂)₉-CH(CH₃-(CH₂)₇)-CH₂-O-(G_{A})₁-H, a₂% molaire du composé de formule CH₃-(CH₂)₉-CH(CH₃-(CH₂)₇)-CH₂-O-(G_{A})₂-H, a₃% molaire du composé de formule CH₃-(CH₂)₉-CH(CH₃-(CH₂)₇)-CH₂-O-(G_{A})₃-H, a₄% molaire du composé de formule CH₃-(CH₂)₉-CH(CH₃-(CH₂)₇)-CH₂-O-( G_{A})₄-H, et a₅% molaire du composé de formule CH₃-(CH₂)₉-CH(CH₃-(CH₂)₇)-CH₂-O-(G_{A})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

12. Utilisation selon la revendication 11, d'un mélange (M_{A}) consistant en, pour 100% de sa masse, de 10,0% massique à 50,0% massique de ladite composition (C_{A}) et de 50,0% massique à 90,0% massique dudit alcanol de formule (IV_{A}).

13. Utilisation selon l'une quelconque des revendications 9 ou 10, **caractérisée en ce que** ledit alcanol de formule (IV) ou ledit mélange d'alcanols de formules (IV), est un mélange d'alcanols de formules (IV_{B}) R_{1B}-OH dans lesquelles le radical R_{1B} représente un radical alkyle linéaire comportant de vingt à vingt-deux atomes de carbone, et ladite composition (C) est une composition (C_{B}) d'alkylpolyglucosides de formules (V_{B}) R_{1B}-O-(G_{B})ₓ-H, dans laquelle le radical R_{1B} est tel que défini ci-dessus, G_{B} représente le radical glucosyle ou α, β -D-glucopyranosyle et x représente un nombre décimal compris entre 1,05 et 2,05, ladite composition (C_{B}) consistant, pour 100% molaire, en un mélange de a₁% molaire du composé de formule R_{1B}-O-(G_{B})₁-H, a₂% molaire du composé de formule R_{1B}-O-(G_{B})₂-H, a₃% molaire du composé de formule R_{1B}-O-(G_{B})₃-H, a₄% molaire du composé de formule R_{1B}-O-(G_{B})₄-H et a₅% molaire du composé de formule R_{1B}-O-(G_{B})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

14. Utilisation selon la revendication 13, d'un mélange (M_{B}) consistant en, pour 100% de sa masse, de 50,0% massique à 90,0% massique d'un mélange d'alcool arachidylique et d'alcool béhénylique et de 10,0% massique à 50,0% massique d'une composition (C_{B}) d'alkyl polyglucosides issue de la réaction en milieu acide du glucose et dudit mélange d'alcool béhénylique et d'alcool arachidylique.

15. Procédé pour réduire ou éliminer l'effet collant ressenti lors de l'application sur la peau d'un gel aqueux comprenant pour 100% de sa masse, de 0,5% massique à 5,0% massique d'un polyélectrolyte anionique réticulé (PA) comportant pour 100% molaire de 5,0% molaire à 95,0% molaire, d'unités monomériques issues de d'acide 2-méthyl-2-((1-oxo-2-propènyl) amino)-1-propanesulfonique de formule (I) (CH₂=CH-C(=O)-NH)-C(CH₃)₂-S(=O)₂-OH, partiellement ou totalement salifié sous forme de sel alcalin ou de sel d'ammonium ; de 4,9% molaire à 90,0% molaire d'unités monomériques issues d'un monomère de formule (II) CH₂=CH-C(=O)-N(R₄)₂, dans laquelle R₄ représente un radical alkyl linéaire ou ramifié comportant de un à quatre atomes de carbone ; et de 0,1% molaire à 5,0% molaire d'unités monomériques issues d'un monomère de formule (III) CH₂=C(CH₃)-C(=O)-O-(CH₂-CH₂-O-)ₙ-R₅, dans laquelle R₅ représente un radical alkyle linéaire ou ramifié comportant de huit à vingt atomes de carbone et n représente un nombre supérieur ou égal à 1 et inférieur ou égal à 20, **caractérisé en ce que** l'on modifie la composition de ce gel aqueux, avant son application sur la peau, en y ajoutant, soit un alcanol de formule (IV) R₁-OH, dans laquelle R₁ représente un radical alkyle linéaire ou ramifié comportant de huit à vingt-deux atomes de carbone, ou un mélange d'alcanols de formules (IV) soit une composition (C) d'alkyl polyglycosides représentés par les formules (V) R₁-O-(G)ₓ-H, dans lesquelles G représente ou bien un reste du glucose ou bien un reste du xylose et x représente un nombre décimal compris entre 1,05 et 2,05, ladite composition (C) consistant, pour 100% molaire, en un mélange de a₁% molaire du composé de formule R₁-O-(G)₁-H, a₂% molaire du composé de formule R₁-O-(G)₂-H, a₃% molaire du composé de formule R₁-O-(G)₃-H, a₄% molaire du composé de formule R₁-O-(G)₄-H, et a₅% molaire du composé de formule R₁-O-(G)₅-H, tel que la somme a₁% + a₂% + a₃% +a₄% + a₅% est égale à 100% molaire et que la somme a₁% + 2a₂% + 3a₃% + 4a₄% + 5a₅% est égale à 100x; soit un mélange (M) dudit alcanol de formule (IV) ou dudit mélange d'alcanols de formules (IV) et de ladite composition (C), en une proportion telle que le rapport massique (R_{M}) au sein du gel aqueux ainsi modifié, ayant comme numérateur ou bien la masse dudit alcanol de formule (IV) ou dudit mélange d'alcools de formules (IV), ou bien la masse de ladite composition (C), ou bien la masse dudit mélange (M) et comme dénominateur, la masse dudit polyélectrolyte anionique réticulé (PA), est supérieur ou égal à 1,0 et inférieur ou égal à 10,0 et plus particulièrement, supérieur ou égal à 1,5 et inférieur ou égal à 5,0.

16. Procédé selon la revendication 15, **caractérisé en ce que** ledit polyélectrolyte anionique réticulé (PA) comporte pour 100% molaire, de 60,0% molaire à 80,0% molaire d'unités monomérique issues de l'acide de formule (I), partiellement salifié sous forme de sel d'ammonium, de 15,0% molaire à 39,5% molaire d'unités monomériques issues du N,N-diméthyl acrylamide, et de 0,5% molaire à 5,0% molaire d'unités monomériques issues du méthacrylate de lauryle tétra-éthoxylé.

17. Procédé selon l'une quelconque des revendications 15 ou 16, **caractérisé en ce que** ledit alcanol de formule (IV) est le 2-octyl dodécan-1-ol de formule (IV_{A}) CH₃-(CH₂)₉-CH(CH₃-(CH₂)₇)-CH₂-OH et ladite composition (C) est une composition (C_{A}) d'alkylpolyxylosides de formules (V_{A}) CH₃-(CH₂)₉-CH(CH₃-(CH₂)₇)-CH₂-O-(G_{A})ₓ-H, dans laquelle G_{A}.représente le radical xylosyle ou α,β-D-xylopyranosyle et x représente un nombre décimal compris entre 1,05 et 2,05, ladite composition (C_{A}) consistant, pour 100% molaire, en un mélange de a₁% molaire du composé de formule CH₃-(CH₂)₉-CH(CH₃-(CH₂)₇)-CH₂-O-(G_{A})₁-H, a₂% molaire du composé de formule CH₃-(CH₂)₉-CH(CH₃-(CH₂)₇)-CH₂-O-(G_{A})₂-H, a₃% molaire du composé de formule CH₃-(CH₂)₉-CH(CH₃-(CH₂)₇)-CH₂-O-(G_{A})₃-H, a₄% molaire du composé de formule CH₃-(CH₂)₉-CH(CH₃-(CH₂)₇)-CH₂-O-( G_{A})₄-H, et a₅% molaire du composé de formule CH₃-(CH₂)₉-CH(CH₃-(CH₂)₇)-CH₂-O-(G_{A})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

18. Procédé selon la revendication 17, **caractérisé en ce que** l'on modifie la composition dudit gel aqueux avant son application sur la peau, en y ajoutant un mélange (M_{A}) consistant en, pour 100% de sa masse, de 10,0% massique à 50,0% massique de ladite composition (C_{A}) et de 50,0% massique à 90,0% massique dudit alcanol de formule (IV_{A}), en une proportion telle qu'au sein dudit gel aqueux ainsi modifié, le rapport massique (R_{MA}) ayant comme numérateur la masse dudit mélange (M_{A}) et comme dénominateur, la masse dudit polyélectrolyte anionique réticulé (PA), est supérieur ou égal à 1,0 et inférieur ou égal à 10,0 et est plus particulièrement supérieur ou égal à 1,5 et inférieur ou égal à 5,0.

19. Procédé selon l'une quelconque des revendications 15 ou 16, **caractérisé en ce que** ledit alcanol de formule (IV) ou ledit mélange d'alcanols de formules (IV), est un mélange d'alcanols de formules (IV_{B}) R_{1B}-OH dans lesquelles le radical R_{1B} représente un radical alkyle linéaire comportant de vingt à vingt-deux atomes de carbone, et ladite composition (C) est une composition (C_{B}) d'alkylpolyglucosides de formules (V_{B}) R_{1B}-O-(G_{B})ₓ-H, dans lesquelles le radical R_{1B} est tel que défini ci-dessus, G_{B} représente le radical glucosyle ou α,β-D-glucopyranosyle et x représente un nombre décimal compris entre 1,05 et 2,05, ladite composition (C_{B}) consistant pour 100% molaire, en un mélange de a₁% molaire du composé de formule R_{1B}-O-(G_{B})₁-H, a₂% molaire du composé de formule R_{1B}-O-(G_{B})₂-H, a₃% molaire du composé de formule R_{1B}-O-(G_{B})₃-H, a₄% molaire du composé de formule R_{1B}-O-(G_{B})₄-H et a₅% molaire du composé de formule R_{1B}-O-(G_{B})₅-H, tel que la somme a₁% + a₂% + a₃% +a₄% + a₅% est égale à 100% molaire et que la somme a₁% + 2a₂% + 3a₃% + 4a₄% + 5a₅% est égale à 100x.

20. Procédé selon la revendication 19, **caractérisé en ce que** l'on modifie la composition dudit gel aqueux, avant son application sur la peau, en y ajoutant un mélange (M_{B}) consistant en, pour 100% de sa masse, de 50,0% massique à 90,0% massique d'un mélange d'alcool arachidylique et d'alcool béhénylique et de 10,0% massique à 50,0% massique d'une composition (C_{B}) d'alkyl polyglucosides issue de la réaction en milieu acide du glucose et dudit mélange d'alcool béhénylique et d'alcool arachidylique, en une proportion telle qu'au sein dudit gel aqueux ainsi modifié, le rapport massique (R_{MB}) ayant comme numérateur la masse dudit mélange (M_{B}) et comme dénominateur, la masse dudit polyélectrolyte anionique réticulé (PA), est supérieur ou égal à 1 et inférieur ou égal à 10 et est plus particulièrement supérieur ou égal à 1,5 et inférieur ou égal à 5,0.
